(19) Europäisches Patentamt
European Patent Office
Office européen des brevets

(11) Veröffentlichungsnummer: **0 276 375 B1**

(12) # EUROPÄISCHE PATENTSCHRIFT

(45) Veröffentlichungstag der Patentschrift: **06.05.92**

(51) Int. Cl.[5]: **C12P 41/00**, C12P 13/00, C12N 9/88

(21) Anmeldenummer: **87115795.4**

(22) Anmeldetag: **28.10.87**

(54) **Verfahren zur Herstellung von optisch aktiven Cyanhydrinen.**

(30) Priorität: **20.01.87 DE 3701383**

(43) Veröffentlichungstag der Anmeldung:
**03.08.88 Patentblatt 88/31**

(45) Bekanntmachung des Hinweises auf die Patenterteilung:
**06.05.92 Patentblatt 92/19**

(84) Benannte Vertragsstaaten:
**AT BE CH DE ES FR GB IT LI LU NL SE**

(56) Entgegenhaltungen:

**CHEMICAL ABSTRACTS, Band 76, Nr. 21, 22. Mai 1972, Seite 196, Zusammenfassung Nr. 123321g, Columbus, Ohio, US; E. GERSTNER et al.: "Flavoenzyme hydroxynitrile lyase (D-oxynitrilase)", & HOPPE-SEYLER'S Z. PHYSIOL. CHEM. 1972, 353(3), 271-86**

**BIOLOGICAL ABSTRACTS, Band 70, Nr. 7, 1980, Seite 4906, Zusammenfassung Nr. 46681, Philadelphia, PA, US; J.M. SCHUMAN: "Studies on the kinetics of cyanohydrin synthesis and cleavage by the flavoenzyme oxynitrilase", & BIOCHIM. BIOPHYS. ACTA 613(1): 203-209. 1980**

**CHEMICAL ABSTRACTS, Band 107, Nr. 1, 6. Juli 1987, Seite 340, Zusammenfassung Nr. 3690p, Columbus, Ohio, US; F. EFFENBERGER et al.: "Enzyme-catalyzed cyanohydrin preparation in organic solvents", & ANGEW. CHEM. 1987, 99(5), 491-2**

(73) Patentinhaber: **Degussa Aktiengesellschaft**
**Weissfrauenstrasse 9**
**W-6000 Frankfurt am Main 1(DE)**

(72) Erfinder: **Effenberger, Franz, Prof. Dr.**
**Schatzweg 5**
**W-7000 Stuttgart 70(DE)**
Erfinder: **Ziegler, Thomas, Dr.**
**Uhlandstrasse 24**
**W-7000 Stuttgart 1(DE)**
Erfinder: **Förster, Siegfried**
**Stubaier Strasse 18**
**W-7000 Stuttgart 60(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(-/2.18/2.0)

## Beschreibung

Die Erfindung betrifft ein Verfahren zur Herstellung von optisch aktiven Cyanhydrinen durch Umsetzung von Aldehyden mit Blausäure in Gegenwart von D-Oxynitrilase.

Optisch aktive Cyanhydrine dienen hauptsächlich als Zwischenprodukte für die Herstellung von entsprechenden optisch aktiven 2-Hydroxycarbonsäuren. Solche optisch aktive 2-Hydroxycarbonsäuren ihrerseits werden in großem Ausmaß mit Chrysanthemumsäure verestert und führen dann zu den wirtschaftlich bedeutenden Pyrethroid-Insektiziden. Optisch aktive Mandelsäure wird daneben häufig zur Spaltung von Racematen basischer Verbindungen eingesetzt.

Aus Biochemische Zeitschrift 346, 301-321 (1966) ist es bereits bekannt, optisch aktive Cyanhydrine durch Umsetzung von Aldehyden mit Blausäure in Gegenwart von D-Oxynitrilase [E.C.4.1.2.10] in einem 0,05 M 50%igen alkoholischen Acetatpuffer für pH 5,4 herzustellen.

Aus der auf die gleichen Autoren zurückgehenden DE-PS 1 300 III ist es ferner bereits bekannt, diese Umsetzung in einem rein wäßrigen 0,05 M Acetatpuffer für pH 5,4 vorzunehmen und die D-Oxynitrilase in Form ihrer Verbindung mit einem im Reaktionsgemisch unlöslichen organischen Ionenaustauscher, wie ECTEOLA-Cellulose (Reaktionsprodukt aus Epichlorhydrin Triäthanolamin und Natriumcellulose) oder DEAE-cellulose (Diethylaminoethyl-Cellulose), einzusetzen.

Durch das bekannte Verfahren, das in einem Reaktionsmedium mit hohem Wassergehalt durchgeführt wird, können zahlreiche Aldehyde in mehr oder weniger hoher chemischer Ausbeute und in mehr oder weniger hoher optischer Ausbeute in optisch aktive Cyanhydrine umgewandelt werden. Der Tabelle 2 auf Seite 310 der oben genannten Literaturstelle "Biochemische Zeitschrift 346" ist jedoch zu entnehmen, daß zahlreiche andere Aldehyde nicht in optisch aktive Cyanhydrine übergeführt werden können.

Das erfindungsgemäße Verfahren ist nun dadurch gekennzeichnet, daß man die Umsetzung in einem mit Wasser nicht mischbaren, aber mit Wasser oder einer wäßrigen Pufferlösung gesättigten organischen Lösungsmittel vornimmt.

Aldehyde, die nach dem bekannten Verfahren in optisch aktive Cyanhydrine umgewandelt werden können, lassen sich durch das erfindungsgemäße Verfahren mit vergleichbarem oder sogar besserem Ergebnis umsetzen. Darüber hinaus aber können nach dem erfindungsgemäßen Verfahren auch solche Aldehyde in optisch aktive Cyanhydrine übergeführt werden, bei denen dies durch das bekannte Verfahren nicht möglich ist.

Das erfindungsgemäße Verfahren ist besonders geeignet für die Umsetzung von Aldehyden der allgemeinen Formel

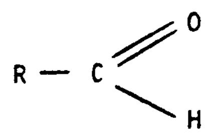 ,

in der R einen der folgenden Reste bedeutet: Methyl, Ethyl, Propyl, i-Propyl, Butyl, i-Butyl, t-Butyl, Pentyl, Hexyl, 2-Methylbutyl, Heptyl, Nonyl, Undecyl, 1-Chlor-1-methyl-ethyl, Oxiranyl, Vinyl, Propenyl, Propen-2-yl, 2-Penten-2-yl, 3-Hepten-3-yl, Penta-1,3-dienyl, 2,6-Dimethyl-penta-1,5-dienyl, 2,6-Dimethyl-penta-5-enyl, 2-Phenylethyl, 1-Phenylethyl, 2-Phenylethenyl, 2-(o-Nitrophenyl)-ethenyl, Cyclohexen-4-yl, Cyclohexyl, Phenyl, o-Methylphenyl, m-Methylphenyl, p-Methylphenyl, o-Chlorphenyl, m-Chlorphenyl, p-Chlorphenyl, o-Nitrophenyl, p-Nitrophenyl, m-Methoxyphenyl, p-Methoxyphenyl, m-Phenoxyphenyl, p-Phenoxyphenyl, o-Phenoxyphenyl, o-Hydroxyphenyl, m-Hydroxyphenyl, p-Hydroxyphenyl, p-Isopropylphenyl, 3-Methoxy-4-hydroxyphenyl, 3,4-Dichlorphenyl, 2,4-Dichlorphenyl, 2,6-Dichlorphenyl, 2,3-Dimethoxyphenyl, 2,4-Dimethoxyphenyl, 2,5-Dimethoxyphenyl, 3,4-Dimethoxyphenyl, 3,4-Methylendioxyphenyl, 2,4-Dihydroxyphenyl, p-Dimethylaminophenyl, $\alpha$-Naphthyl, 2-Pyrrolyl, 2-Furyl, 2-Thienyl, 3-Thienyl, 2-Pyridyl, 3-Pyridyl, 4-Pyridyl, Chlormethyl, Dichlormethyl, Trichlormethyl, Fluormethyl, Difluormethyl, Trifluormethyl oder 2-Methylmercaptoethyl.

Die Bläusäure wird in einer dem eingesetzten Aldehyd mindestens äquivalenten Menge eingesetzt. Vorteilhafter ist jedoch die Anwendung eines Überschusses bis zu etwa 5 Moläquivalenten.

D-Oxynitrilase kann aus käuflicher Bittermandelkleie gewonnen werden. Sie kann nach Fällung mit beispielsweise Ammoniumsulfat als freies Enzym eingesetzt werden, das im Reaktionsgemisch suspendiert wird.

Vorteilhafterweise wird dann die Umsetzung in einem Enzym-Membran-Reaktor vorgenommen. Vorzugsweise aber wird die D-Oxynitrilase in immobilisierter Form eingesetzt, beispielsweise auf Glaskügelchen, Ce-jonenaustauscher, DEAE-Sephadex und insbesondere auf AVICEL-cellulose oder ECTEOLA-Cellulose.

Für das erfindungsgemäße Verfahren geeignete organische Lösungsmittel sind beispielsweise aliphatische Kohlenwasserstoffe, wie Petrolether, Hexan oder Cyclohexan, aromatische Kohlenwasserstoffe, wie Benzol oder Toluol, chlorierte aliphatische Kohlenwasserstoffe, wie Methylenchlorid,

Chloroform oder Trichlorethan, Ether, wie Diethylether, Diisopropylether oder t-Butylmethylether, Carbonsäurealkylester, wie Ester der Ameisensäure, Essigsäure, Propionsäure, Buttersäure, Pentancarbonsäure und Hexancarbonsäure mit Methyl-, Ethyl-, n-Propyl-, i-Propyl-, n-Butyl-, i-Butyl-, t-Butyl-, n-Pentyl-und n-Hexylalkohol, und aliphatische oder cycloaliphatische Alkohole, wie n-Butanol, n-Pentanol, n-Hexanol, n-Octanol oder Cyclohexanol.

Das organische Lösungsmittel wird mit Wasser oder vorzugsweise mit einer wäßrigen Pufferlösung gesättigt, insbesondere einer solchen für den pH-bereich zwischen 3 und 8. Geeignete Pufferlösungen sind beispielsweise 0,001 - 1,0 molare Lösungen von Natriumacetat und Essigsäure, Natrium- oder Kalium-dihydrogenphosphat mit Natrium- oder Kaliumhydroxidlösung oder Phosphorsäure, Tris-(hydroxymethyl)-aminomethan und Salzsäure, Borsäure und Natriumhydroxid, Citronensäure und Natriumhydroxid.

Die Umsetzung des Aldehyds mit der Blausäure kann bei einer Temperatur zwischen 0 und 90 ° C vorgenommen werden, vorzugsweise bei Raumtemperatur.

Die durch das erfindungsgemäße Verfahren erhältlichen rohen Lösungen der optisch aktiven Cyanhydrine sind überraschenderweise gut haltbar. Sie müssen nicht unbedingt aufgearbeitet werden, sondern können unter Umständen direkt weiterverarbeitet werden, z.B. durch Hydrolyse zu den entsprechenden optisch aktiven 2-Hydroxycarbonsäuren.

Wenn die D-Oxynitrilase in immobilisierter Form eingesetzt werden soll, wird sie als wäßrige Lösung auf das Trägermaterial aufgebracht. Nach Trocknung an der Luft ist das immobilisierte Enzym als Katalysator einsatzbereit.

Die praktische Durchführung des erfindungsgemäßen Verfahrens kann beispielsweise so erfolgen, daß das Enzym oder die damit hergestellte Katalysatorzubereitung in Essigsäureethylester, der mit einem 0,01 M Acetatpuffer für pH 5,4 gesättigt ist, suspendiert bzw. aufgeschlämmt wird, und daß die Suspension bzw. Aufschlämmung dann mit dem Aldehyd und schließlich mit der Blausäure, gegebenenfalls als Lösung in Essigsäureethylester, versetzt wird. Nach beendeter Umsetzung werden die ungelösten Bestandteile des Reaktionsgemisches abfiltriert und das Filtrat wird, beispielsweise über Natriumsulfat, getrocknet. Nach erneuter Filtration und Abdampfen des Lösungsmittels unter vermindertem Druck hinterbleibt das optisch aktive Cyanhydrin als farbloses bis blaßgelbes öl.

Die Erfindung wird durch die nachfolgenden Beispiele näher erläutert. Zur exakten Bestimmung der Enantiomerausbeute wird eine Probe des gebildeten Cyanhydrins mittels R(+)-Methoxytrifluormethylphenylacetylchlorid in den [ R(+)-MTPA] -Ester übergeführt und dieser durch Kapillargaschromatographie in die beiden Diastereomeren aufgetrennt. Angegeben ist jeweils der Diastereomerenüberschuß in %.

Beispiel 1:

2,0 g AVICEL-Cellulose (Hersteller: Merck, Darmstadt) wurden in 20 ml wäßrigem 0,01 M Acetatpuffer für pH 5,4 2 Stunden lang gequollen, dann abgesaugt und abgepreßt. Nach erneutem Aufschlämmen in 50 ml des gleichen Puffers wurden bei Raumtemperatur unter Umschwenken 150 µl einer D-Oxynitrilase-Lösung mit einem Gehalt von 700 Units D-Oxynitrilase/ml ($A_{sp}$ = 65 Units/mg) in 0,020 M Acetatpuffer für pH 5,4 zugetropft. Es wurde 1 Stunde lang bei Raumtemperatur gerührt, dann wurde die imprägnierte Cellulose abgetrennt, abgepreßt und getrocknet.

Die Katalysatorzubereitung wurde in 25,0 ml mit 0,01 M Acetatpuffer für pH 5,4 gesättigtem Essigsäureethylester suspendiert und die Suspension wurde nacheinander mit 0,53 g (5 mMol) Benzaldehyd und 250 µl (6,5 mMol) Blausäure versetzt. Es wurde 2,5 Stunden lang bei Raumtemperatur gerührt, dann wurde der Katalysator abfiltriert und das Filtrat über Natriumsulfat getrocknet. Nach Filtration und Abdampfen des Lösungsmittels unter vermindertem Druck hinterblieben 0,63 g (95 % der Theorie) Mandelsäurenitril als farbloses öl.

$[\alpha]_D^{20}$ = + 49° (c = 5, $CHCl_3$), Diastereomerenüberschuß: 99,3 %.

$^1$H-NMR ($CDCl_3$): 7,50 (s, $H^{arom}$, 5H), 5,50 (s,-CH-CN, 1H), 3,78 ($\bar{s}$, OH, 1H).

Beispiel 2:

5,0 g ECTEOLA-Cellulose (Hersteller: Macherey, Nagel u. Co, Düren) wurden in 150 ml wäßrigem 0,01 M Acetatpuffer für pH 5,4 2 Stunden lang gequollen, dann abgesaugt und abgepreßt. Nach erneutem Aufschlämmen in 50 ml des gleichen Puffers wurde bei Raumtemperatur unter Umschwenken eine D-Oxynitrilase-Lösung (1,5 mg D-Oxynitrilase, $A_{sp}$ = 60 Units/mg, in 0,5 ml 0,025 M Phosphatpuffer für pH 7,0) zugetropft. Es wurde 1 Stunde lang bei Raumtemperatur gerührt, dann wurde die imprägnierte Cellulose abgetrennt, abgepreßt und getrocknet.

Die Katalysatorzubereitung wurde in 40 ml mit 0,01 M Acetatpuffer für pH 5,4 gesättigtem Essigsäureethylester suspendiert und die Suspension wurde nacheinander mit 1,36 g (10 mMol) m-Methoxybenzaldehyd und 1 ml eines Gemisches aus Blausäure und Essigsäureethylester im Volumenverhältnis 1:1 (13 mMol HCN) versetzt. Es wurde 1,5 Stunden lang bei Raumtemperatur gerührt, dann wurde der Katalysator abfiltriert und das Filtrat über Natriumsulfat getrocknet. Nach Filtration und Abdampfen des Lösungsmittels unter vermindertem Druck hinterblieben 1,55 g (95 % der Theorie) m-Methoxymandelsäurenitril als gelbliches öl.

$[\alpha]_D^{20}$ = + 33,0° (c = 5, $CHCl_3$)

Diastereomerenüberschuß: 90 %.
$^1$H-NMR($CDCl_3$): 6,82-7,50 (m, $H^{arom}$, 4H), 3,82 (s,$OCH_3$, 3H), 5,50($\overline{s}$, OH, 1H), 4,05 ($\overline{s}$, OH, 1H).

Beispiel 3:

Entsprechend dem Beispiel 1 wurden 0,99 g (5 mMol) m-Phenoxybenzaldehyd in 50 ml Reaktionsmedium in einer Reaktionszeit von 8 Tagen umgesetzt. Nach der Aufarbeitung hinterblieben 1,12 g (99 % der Theorie m-Phenoxymandelsäurenitril als gelbliches öl.

$[\alpha]_D^{20}$ = + 6° (c= 5, $CHCl_3$), Diastereomerenüberschuß: 98 %.

$^1$H-NMR ($DCDl_3$): 7,60 - 6,90 (m, $H^{arom}$, 9H), 5,50 (s,-CH-CN, 1H), 3,95 ($\overline{s}$, OH, 1H).

Beispiel 4:

Entsprechend dem Beispiel 1 wurden 0,48 g (5 mMol) Furfural in einer Reaktionszeit von 4 Stunden umgesetzt. Nach der Aufarbeitung hinterblieben 0,54 g (88 % der Theorie) α-Hydroxy-(2-furyl)-acetonitril als gelbliches öl.

$[\alpha]_D^{20}$ = + 50,3° (c = 5,$CHCl_3$), Diastereomerenüberschuß: 98,5 %.

$^1$H-NMR ($CDCl_3$): 6,60-6,30 (m, $H^{furan}$, 2H) 7,42 (mc, $H^{furan}$, 1H), 5,50 (s,-CH-CN, 1H), 4,00 ($\overline{s}$, OH, 1H).

Beispiel 5:

Entsprechend dem Beispiel 2 wurden 1,32 g (10 mMol) Zimtaldehyd in einer Reaktionszeit von 11 Tagen umgesetzt. Nach der Aufarbeitung hinterblieben 1,48 g (93 % der Theorie) 2-Hydroxy-4-phenyl-3-butensäurenitril als weißer Feststoff. Diastereomerenüberschuß: 45 %.

$[\alpha]_D^{20}$ = + 11,3° (c = 5, $CHCl_3$),

Schmelzpunkt: 65 - 70°C.
Nach Umkristallisation aus n-Hexan:

$[\alpha]_D^{20}$ = + 3,7° (c = 5, $CHCl_3$),

Diastereomerenüberschuß: 31 %.
Schmelzpunkt: 76-77°C.
$^1$H-NMR ($CDCl_3$): 7,47 ($\overline{s}$, $H^{arom}$ 5H), 6,45 (d, Ph-CH=, 1H), 6,20 ($\overline{d}$ = CH-CH-CN, 1H), 5,25 ($\overline{d}$,-CH-CN, 1H), 3,80 ($\overline{s}$, OH, 1$\overline{H}$)
Literatur:
Schmelzpunkt: 75°C. [ G. Peine, Chem. Ber. 17, 2113 (1884)].

Beispiel 6:

Entsprechend dem Beispiel, 1 wurden 0,35 g (5 mMol) Crotonaldehyd in einer Reaktionszeit von 3 Stunden umgesetzt. Nach der Aufarbeitung hinterblieben 0,33 g ( 68 % der Theorie) 2-Hydroxy-3-pentensäurenitril als gelbliches öl.

$[\alpha]_D^{20}$ = 24,7° (c = 5, $CHCl_3$),

Diastereomerenüberschuß: 97 %.
$^1$H-NMR($CDCl_3$): 6,30-5,40 (m,=CH, 2H), 4,95 (d,-CH-CN, 1H), 3,50 (s, OH, 1H), 1,80 (d, $CH_3$, 3H).

Beispiel 7:

Entsprechend dem Beispiel 2 wurden 0,43 g (5 mMol) Pivalaldehyd in einer Reaktionszeit von 4,5 Stunden umgesetzt. Nach der Aufarbeitung hinterblieben 0,44 g (78 % der Theorie) 3,3-Dimethyl-2-hydroxy butansäurenitril als farbloses öl.

$[\alpha]_D^{20}$ = + 11,1° (c = 5, $CHCl_3$),

Diastereomerenüberschuß: 73 %.
$^1$H-NMR ($CDCl_3$): 4,14 (s,-CH-CN, 1H), 3,25 ($\overline{s}$, OH, 1H), 1,10 (s, $CH_3$, 9H).

Beispiel 8:

Entsprechend dem Beispiel 1 wurden 0,60 g (5 mMol) Phenylacetaldehyd in einer Reaktionszeit von 4,5 Stunden umgesetzt. nach der Aufarbeitung hinterblieben 0,70 g (95 % der Theorie) Phenylmilschsäurenitril als gelbliches öl.

$[\alpha]_D^{20}$ = + $2,0^0$ (c = 5, $CHCl_3$),

Diastereomerenüberschuß: 40 %.
$^1$H-NMR ($CDCl_3$) 7,37 (s, $H^{arom}$, 5H), 4,60 (t,-CH-CN, 1H), 3,40 ($\overline{s}$, OH, 1H), 3,10 (d, $CH_2$, 2H)

Beispiel 9:

Entsprechend dem Beispiel 1 wurden 0,52 g (5 mMol) 3-Methylmercapto-propionaldehyd in einer Reaktionszeit von 6,5 Stunden umgesetzt. Nach

der Aufarbeitung hinterblieben 0,64 g (97 % der Theorie) 2-Hydroxy-4-methylmercapto-buttersäurenitril als gelbliches öl.

$[\alpha]_D^{20}$ = + 21,5 ° (c = 5, $CHCl_3$),

Diastereomerenüberschuß: 80 %.
$^1$H-NMR ($CDCl_3$): 4,80 (t,-CH-CN, 1H), 3,72 ($\bar{s}$, OH, 1H), 2,80 (dt, $CH_2$-CH-CN, 2H), 2,29 (t,-S-$CH_2$-, 2H), 2,22 (s, $H_3C$-$\bar{S}$-, 3H).

Beispiel 10:

Entsprechend dem Beispiel 1 wurden 0,28 g (5 mMol) Acrolein in einer Reaktionszeit von 6 Stunden umgesetzt. Nach der Aufarbeitung hinterblieben 0,24 g (58 % der Theorie)2-Hydroxybut-3-ensäurenitril als farbloses öl.

$[\alpha]_D^{20}$ = -0,5 ° (c = 5, $CHCl_3$),

Diastereomerenüberschuß: 12 %.
$^1$H-NMR ($CDCl_3$): 6,34-5,40 (m, = CH,3H), 5,10 (d,-CH-CN,1H), 3,68 ($\bar{s}$, OH, 1H).

Beispiel 11:

Entsprechend dem Beispiel 1 wurden 0,36 g (5 mMol) Butyraldehyd in einer Reaktionszeit von 4,5 Stunden umgesetzt. Nach der Aufarbeitung hinterblieben 0,37 g (75 % der Theorie) 2-hydroxypentansäurenitril als farbloses öl.

$[\alpha]_D^{20}$ = + 13,1 ° (c = 5, $CHCl_3$),

Diastereomerenüberschuß: 96 %.
$^1$H-NMR ($CDCl_3$): 1,2 (d, $CH_3$, 3H), 1,6 (m, $CH_2$, 4H), 4,6 (t, CH, 1H), 4,0 ($\bar{s}$, CH, 1H).

**Patentansprüche**

1. Verfahren zur Herstellung von optisch aktiven Cyanhydrinen durch Umsetzung von Aldehyden mit Blausäure in Gegenwart von D-Oxynitrilase, dadurch gekennzeichnet, daß man die Umsetzung in einem mit Wasser nicht mischbaren, aber mit Wasser oder einer wäßrigen Pufferlösung gesättigten organischen Lösungsmittel vornimmt.

2. Verfahren nach Anspruch 1, dadurch gekennzeichnet, daß man als organisches Lösungsmittel einen aliphatischen oder aromatischen Kohlenwasserstoff, einen chlorierten aliphatischen Kohlenwasserstoff, einen Ether, einen Carbonsäurealkylester oder einen aliphatischen oder cycloaliphatischen Alkohol einsetzt.

3. Verfahren nach Anspruch 1 oder 2, dadurch gekennzeichnet, daß das organische Lösungsmittel mit einer wäßrigen Pufferlösung für den pH-Bereich zwischen 3 und 8 gesättigt ist.

**Claims**

1. A process for the preparation of optically active cyanohydrins by the reaction of aldehydes with hydrocyanic acid in the presence of D-oxynitrilase, characterised in that the reaction is carried out in an organic solvent which is immiscible with water but saturated with water or an aqueous buffer solution.

2. A process according to Claim 1, characterised in that the organic solvent used is an aliphatic or aromatic hydrocarbon, a chlorinated aliphatic hydrocarbon, an ether, a carboxylic acid alkyl ester or an aliphatic or cycloaliphatic alcohol.

3. A process according to Claim 1 or 2, characterised in that the organic solvent is saturated with an aqueous buffer solution for the pH range of from 3 to 8.

**Revendications**

1. Procédé d'obtention de cyanhydrines optiquement actives par réaction d'aldéhydes avec l'acide cianhydrique en présence de D-oxynitrilase, caractérisé en ce que l'on entreprend la réaction dans un solvant organique non miscible à l'eau, mais saturé avec de l'eau ou avec une solution aqueuse tampon.

2. Procédé selon la revendication 1, caractérisé en ce que l'on met en oeuvre comme solvant organique un hydrocarbure aliphatique ou aromatique, un hydrocarbure aliphatique chloré, un éther, un ester alcoylique d'acide carboxylique, ou un alcool aliphatique ou cycloaliphatique.

3. Procédé selon la revendication 1 ou 2, caractérisé en ce que le solvant organique est saturé avec une solution aqueuse tampon pour une zone de pH comprise entre 3 et 8.